# EUROPEAN PATENT SPECIFICATION

(11) **EP 1 994 912 B1**
(45) Date of publication and mention of the grant of the patent: **03.03.2010**
(21) Application number: 07108552.6
(22) Date of filing: 21.05.2007
(51) Int. Cl.: A61F 2/24, A61M 1/10

(54) **Electromagnetically closable non-return valve for biological liquid pumps**
Elektromagnetisch schließbares Rückschlagventil für biologische Flüssigkeitpumpen
Valve anti-retour à fermeture commandée électromagnétiquement pour une pompe à fluides biologiques

(43) Date of publication of application: 26.11.2008
(73) Proprietor: Della Sala, Berardino, 20122 Milano (IT)
(72) Inventor: Della Sala, Berardino, 20122 Milano (IT)
(74) Representative: Ripamonti, Enrico

(56) References cited:
- WO-A-2005/046467
- FR-A1- 2 485 928
- JP-A- 60 132 081
- US-A- 4 979 955
- US-A- 6 039 759
- US-B1- 6 770 024

## Description

The present invention relates to an electromagnetically closable non-return valve for pumps for biological liquids, in particular blood, the valve being more particularly able to be usefully employed both in an artificial heart and in a patient's heart requiring replacement of one or more heart valves.

As known to the experts of the art, in an artificial heart the same artificial valves are used as those generally used in the clinical sector. They are an element of fundamental importance and their structure conditions the hydraulic characteristics of the blood which passes through them, they being not only able to cause damage to the blood cells and the blood in general, but also influencing the quality of the functional cardiac curves (flow, throw, ejection fraction).

The requirements which these artificial valves should satisfy are:
1) to oppose a hydraulic resistance which is as low as possible to direct flow and a maximum to reverse flow;
2) to have opening and closure times which are short compared with the systole and diastole duration;
3) to have movable elements which do not give rise to vibrations.

In this respect it is known to the experts of the art that in the natural heart, blood refluxes occur through the heart valves (the reflux in the left ventricle being greater than in the right) and that these natural refluxes take place both during the closure stage, to cause these valves to close and also, not rarely, with the valves closed because of their imperfect sealing. These refluxes mean that the effective flow is less than that enabled by the valve ostium.

Various models of artificial hearts have been proposed and utilized in clinical use for treating heart valve pathologies and have also been applied in many cases to artificial heart devices.

The first artificial heart to have been historically used, in the 1970s, is that known as the Starr-Edwards valve, which is essentially a non-return valve with a ball as valving element, and comprises a rigid cage of stellite. This latter, on opening, retains the valving element in the form of a silicone-coated rubber ball. The valve seat is defined by an annular teflon element, which is sutured onto the annulus. Valve closure is caused by the blood reflux, which urges the ball valving element against the relative valve seat or valve ostium.

Valves with a disc valving element (so-called monodisc) were then produced and used, such as the Bijork-Shiley and that known as the Medtronic Hall Valve, the two differing essentially only by the shape of the central support pin for the disc. They comprise a rigid annular element which defines the valve ostium and is lined with biomaterial, and which serves also to fix the valve to the annulus by suture. As stated, the valving element is a rigid disc, free to oscillate about a diametrical axis thereof in order to be able to assume a position of closure of the valve seat and a position of opening. Again in this case the valving element is brought into its closure position by the blood reflux.

A variant of this type of valve is the St. Jude Medical valve, in which the disc valving element is composed of two halves (bi-leaflet) each hinged along the common diameter, so that the two half discs can pass simultaneously from their open position to their closure position by a "butterfly wing" movement, closure again taking place by blood reflux.

More recently two biological valves known respectively as the lonescu Shiley and the Mitroflow of Mitral Medical have been used. These are closest to natural heart valves, being provided with cusp-shaped flexible pockets (normally three in number) which project inwardly from an annular element defining the valve ostium. In closure, the three pockets together assume a swallows nest shape. The pockets are lined with bovine pericardium to make the valve less inclined to form thromboses. However, again in this case the pockets assume their closure position (by a so-called "parachute effect") as a result of blood reflux.

From the aforegoing it is apparent that both natural heart valves and artificial heart valves are of passive operation, and for this reason involve an effective blood flow which is reduced below that which would be allowable by the valve ostium, precisely because of the blood reflux.

Document FR 2 485 928 A1 discloses an artificial heart with an electromagnetically commanded valve, where the valve opening is controlled. Document US 6 770 024 B1 discloses a left ventricle assist device with an active ferromagnetic valve, where the valve closing is controlled.

An object of the present invention as claimed is to provide an artificial valve for biological liquids which is of controllable closure, i.e. is of active operation, so that the valve can be closed before reflux takes place, or in other words valve closure does not take place as a result of reflux, this enabling an effective flow to be obtained which is equal to that allowable by the valve ostium port, hence avoiding the aforedescribed penalization; and more particularly an artificial heart valve, usable both as an independent prosthesis in valvulopathy corrective surgery, and in a complete artificial heart device.

This object is attained by the valve of the present invention as claimed, comprising a substantially rigid annular element of biocompatible material, which encloses the valve ostium, and valve ostium closure means, also of biocompatible material, characterised in that the valve ostium closure means comprise at least two flexible closure flaps, of a biocompatible material, which extend towards the interior of the valve ostium and which simulate the flaps of natural heart valves, the flaps containing in their interior, at least in their distal part, a ferromagnetic fluid, there being provided:
- means for generating a magnetic field which, by acting on the ferromagnetic fluid contained in the closure flaps, causes them to approach each other and consequently close the valve;
- tie means, also of biocompatible material, which simulate the heart valve cords, to retain the distal end of each flap in the closure position;
- pressure sensor means which enable the biological liquid pressure to be measured upstream and downstream of the valve;
- a control unit for activating the magnetic field when the pressure sensors sense that the pressure in the biological liquid upstream and downstream of the valve are equal, and to deactivate it, at the latest, when the valve is to be opened.

Consequently, if a magnetic field is generated which tends to cause mutual approach of the ferromagnetic fluid portions contained in the relative closure flaps, an active closure of the valve is obtained, this closure being able to be effected at that moment in time which is most opportune for the purpose of eliminating the aforestated reflux. The moment in which to effect closure while avoiding reflux is obtainable by said pressure sensor means.

Ferromagnetic fluids have been used for at least two decades in radiodiagnostics as a contrasting medium (see for example "Medical Application of Magnetic Fluid" by P.D.S. Verma, in Acta of Second International Conference on Physiolagical Fluid-dynamics, Madras (India), August 10-12, 1987). These are essentially colloidal suspensions of minuscule ferromagnetic particles in a suitable liquid vector.

A ferromagnetic fluid has already been used by the inventor of the present invention to form an artificial heart (see US 4650485 A), and also a pump for biological liquids (see EP 0272445 B1) also usable as an artificial heart. The valves used in those applications were however of passive type.

If the valve is used in a biological liquid pump of ferromagnetic fluid type, and in particular in an artificial heart of this type, the ferromagnetic fluid contained in the closure flaps of the valve can be made to communicate with the ferromagnetic fluid surrounding the flexible inner pocket of the pump or the corresponding flexible inner pocket of the relative artificial heart ventricle. According to this embodiment of the invention a valve is obtained which is perfectly integrated and coordinated with the pump, or the corresponding artificial heart ventricle, of ferromagnetic fluid type.

The said means for generating the magnetic field causing the valve to close comprise in particular a winding of electrically conductive wire through which it is sufficient to pass direct electric current at the moment in which said magnetic field is to be created. The winding can for example surround the outside of the valve annular element or form part of this latter, the relative turns lying in planes substantially perpendicular to the valve axis.

It should be noted that said tie means, as do the cords of heart valves, enable the valve flaps to be retained in their closure position even if, immediately after valve closure due to the action of the magnetic field, this latter should disappear, this being achieved by virtue of the already stated "parachute effect", which also exists in natural heart valves when the ventricular pressure increases. However it can be convenient to maintain the magnetic field until the moment in which the valve is to open, to ensure a perfect valve seal for the entire closure period.

The active valve of the invention, besides enabling reflux to be eliminated, also enables the following advantages to be obtained:
- for equal valve ostium ports, an increase in throw and actual flow, which coincides with the maximum allowable by the port;
- a clear reduction, if not elimination, of vibrations of the moving valve parts, in particular of its flaps, as the valve can already be in its closure configuration at the moment in which the transvalvular pressure gradient begins to increase;
- consequent clear reduction in the mechanical fatigue on the valve moving parts, with reduction in the risk of breakage by fatigue;
- if the biological liquid is blood, a lesser traumatic effect on its components compared with known artificial valves, and in particular a significant reduction in hemolysis and thrombogenicity, all by virtue of the flexibility of the valve flaps and of the clear reduction in vibrations of these latter.

The invention will be more apparent from the ensuing description of some exemplifying embodiments thereof given by way of example. In this description reference will be made to the accompanying drawings, in which:
Figure 1 is a plan view from above, of a valve according to one embodiment of three flap type, shown in its closure condition;
Figure 2 is an enlarged schematic section therethrough, taken on the line 2-2 of Figure 1, the valve, in the configuration shown in this figure, performing the function of a valve for replacing the mitral valve or the tricuspid of an artificial heart of ferromagnetic fluid type;
Figure 3 is similar to Figure 1, but shows the valve in its condition of maximum opening;
Figure 4 is a section through a pump of ferromagnetic fluid type for biological liquids, with the valve, representing another embodiment, in the closed condition;
Figure 5 is a view from above of just the valve of Figure 4, again in the closed condition, from which it can be seen that the valve is of the type comprising only two flaps.

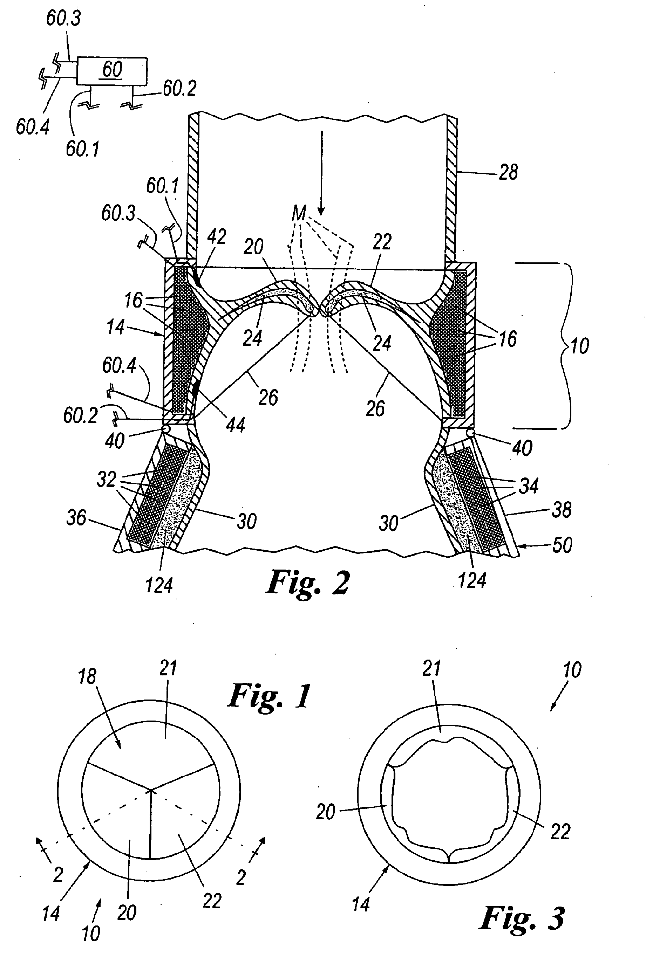

Examining Figures 1-3, it can be seen that the valve 10 is of overall cylindrical shape, comprising a substantially rigid annular element 14, the longitudinal section of which is in the shape of two opposing Cs. The annular element 14 encloses a coaxial winding 16 of electrically conductive wire. The closure means 18 (Figure 1) for the valve 10 are fixed inside the annular element 14, they consisting in this specific case of three flexible flaps, 20, 21 and 22 respectively. These flaps are of a biologically compatible material (for example of nylon, or of those materials indicated by the trademarks Dacron, Teflon or Sylastic, or of those types of polyurethane presenting said characteristics, all materials already used in artificial heart valves or for vascular by-passes). Internally, the three flaps 20-22 present respective closed cavities, indicated by 24, which are filled with a ferromagnetic fluid. As can be seen (Figure 2), this latter mainly occupies the distal end part of the three flaps. As also shown in Figure 2, the distal end of each flap 20-22 is fixed to one end of a substantially inextensible respective tie 26 (the ties 26 being in the traction situation when the valve is in the closed configuration, whereas when in the open configuration they are loose, the other end of the tie 26 being fixed to the bottom of the annular element 14).

The valve 10 of Figure 2 functions as a valve able to replace a mitral or a tricuspid valve, it being upperly connected in known manner (for example by suture) to a vessel 28 and lowerly to a ventricle of an artificial heart of ferromagnetic fluid type, of this ventricle there being visible in Figure 2 the upper part of a flexible internal pocket 30 internally bounding ferromagnetic fluid 124, and also two of the windings 32 and 34 of electrically conductive wire embedded in corresponding rigid outer walls 36 and 38 hinged at 40 to the valve 10. For further clarification of this type of artificial heart, reference should be made to the initially cited prior patents.

In Figure 2 two pressure sensors are visible, of which one, indicated by 42 is located in this specific case on the annular element 14 just upstream, and the other, indicated by 44, is disposed on the same annular element but just downstream. The two sensors 42 and 44 are connected in conventional manner (60.1, 60.2) to a control unit indicated overall by 60, to which the winding 16 is also connected (60.3, 60.4). The control unit 60 can evidently also simultaneously control the other valves of the pump or of the artificial heart. Such a function can be performed by the same control unit of the pump or artificial heart 50.

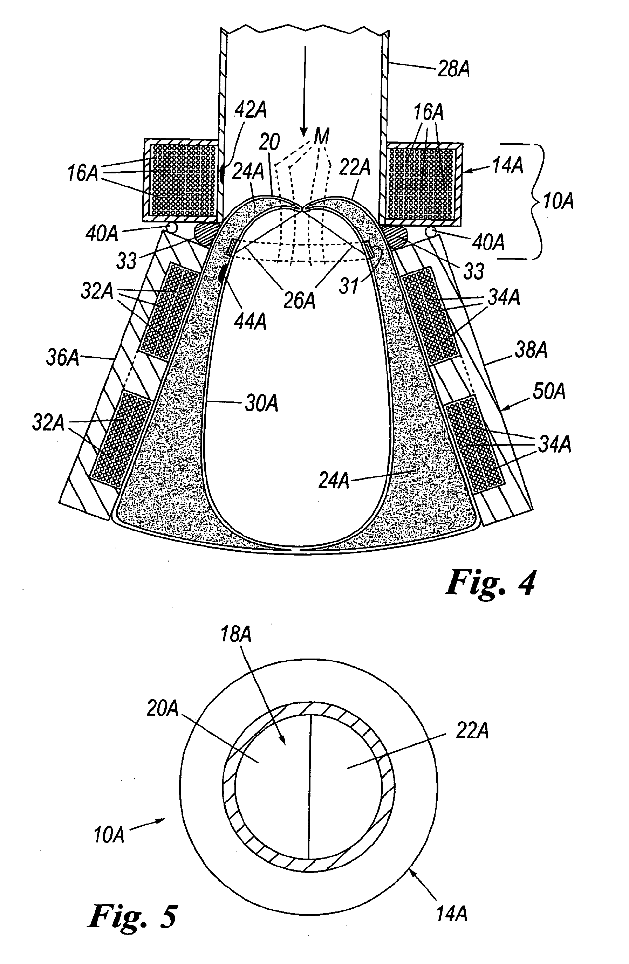

In Figure 4 (in which elements equal or similar to those of Figure 2 have been indicated by the same reference numeral with the addition of A) another embodiment of the valve, indicated by 10A, is shown presenting the characteristic of being more greatly integrated into a biological liquid pump of ferromagnetic fluid type, indicated overall by 50A, which can also be a ventricle of an artificial heart. In this respect, the flaps of the valve 10A are in this specific case only two in number (Figure 5), indicated by 20A and 22A, the ferromagnetic fluid contained in these two flaps communicating with the ferromagnetic fluid bounded by the flexible pocket 30A, to form a single group, so that all the ferromagnetic fluid has been indicated overall by 24A.

In this solution, inside the pocket 30A a rigid ring 31 is provided, to which the lower ends of the two ties 26A are fixed.

An elastic connection and seal ring 33, of biocompatible material, is disposed between the annular element 14A and the pocket 30A, its elasticity enabling the plates 36A and 38A to oscillate.

Returning to the valve 10, its operation should already be apparent for experts of the art, from the aforegoing. However it will be briefly described with reference to Figures 1-3, in which the valve 10 is used as replacement for a mitral or a tricuspid valve.

Starting from open condition of the valve 10 (Figure 3) and by virtue of the fact that it is of active type, by means of the control unit 60 (for example of microprocessor type) connected to the two pressure sensors 42, 44 (Figure 2) and to the winding 16, at the moment in which the pressures on the opposite sides of the valve 10 are equal, direct electric current can be made to pass through the winding 16 in order to generate a magnetic field (of which some lines of force have been partially represented with dashed lines indicated by M) which urges the ferromagnetic fluid 24, contained in the three flaps 20-22 of the valve 10, to migrate rapidly towards the axis of the valve, so that it passes from the open condition of Figure 3 to the closed condition of Figures 1 and 2, this occurring before the intraventricular pressure begins to increase. As the distal ends of the flaps 20-22 of the valve 10 are retained by the respective ties 26, at the moment in which the intraventricular pressure begins to increase (because of contraction of the ventricle 50), the flaps 20-22 having already been brought into mutual contact, the creation of a greater pressure on the ventricular face of the valve 10 (the lower face, with reference to Figure 2) can only cause the seal thereof to increase (for the already stated "parachute effect") even if current passage through the winding 16 ceases. However, as already stated, in order to ensure perfect sealing of the valve 10, it can be decided to maintain current passage through the winding 16 (and hence the magnetic field M) for the entire period in which the valve has to remain closed.

When the ventricle 50 begins to dilate, so that the ventricular pressure becomes less than the pressure of the vessel 28, current passage through the winding 16 having ceased in the meantime, the valve 10 opens automatically, the flaps 20-22 opening downwards (again with reference to Figure 2) to become arranged in accordance with the configuration of Figure 3.

At this point the cycle can be repeated as soon as the pressure on the two sides of the valve returns to zero, or rather just before this happens in order to take account of the time necessary for the flaps 20-22 to assume the closure condition. The control unit (which has not been shown for simplicity in the case of the valve 10A) can consequently be prearranged to activate the magnetic field M with the advance which enables this result to be obtained.

With regard to the valve 10A of Figures 4 and 5, the fact that its closure element 18A comprises only two flaps 20A and 22A instead of the three of the valve 10 does not change its operation, closure being again activated by the passage of direct current through the winding 14A, so as to create a magnetic field the lines of force of which have been partially represented by dashed lines again indicated by M. As can be seen, the valve 10A is again provided with substantially inextensible flexible ties 26 (in this specific case only two) which retain the distal end of the relative flaps 20A, 22A.

Although in the drawings the valves 10 and 10A are shown as forming part of a pump (50 and 50A respectively) of the ferromagnetic fluid type and more particularly of an artificial heart ventricle of this type, it is important to note that these specific valves (and more generally the valves according to the present invention) can be used in artificial hearts of any type, as well as to replace natural heart valves if this should be necessary.

In particular, although in the drawings the valves 10 and 10A have been represented as valves able to replace a mitral valve or a tricuspid valve, the valve of the present invention can be likewise used as an aortic valve or pulmonary valve, for this purpose it being sufficient to simply invert it.

## Claims

1. Non-return valve (10, 10a) for pumps (50; 50A) for biological liquids, comprising a substantially rigid annular element (14; 14A) of biocompatible material, which encloses the valve ostium, and valve ostium closure means (18; 18A), also of biocompatible material, the valve ostium closure means (18; 18a) comprising at least two flexible closure flaps (20-22; 20A, 22A), of a biocompatible material, which extend towards the interior of the valve ostium and which simulate the flaps of natural heart valves, there being provided:
- means (16; 16A) for generating a magnetic field (M);
- substantially inextensible ties (26; 26A), also of biocompatible material,
- pressure sensor means (42, 44) which enable the biological liquid pressure to be measured upstream and downstream of the valve (10; 10A);
- a control unit (60) for activating the magnetic field (M) when the pressure sensors (42, 44) sense that the pressure in the biological liquid upstream and downstream of the valve (10; 10A) are equal, and to deactivate it, the latest, when the valve (10; 10A) is to be opened, **characterised in that** the said flaps (20-22; 20A, 22A) contain in their interior, at least in their distal part, a ferromagnetic fluid (24; 24A), said means for generating a magnetic field by acting on the ferromagnetic fluid (24; 24A) contained in the closure flaps (20-22; 20A, 22A), causes them to mutually approach and consequently close the valve (10; 10A); the distal end of each said flap (20-22; 20A, 22A) is fixed to one end of a respective said tie (26; 26A), the other end of the said tie (26; 26A) is fixed to the bottom of the annular element (14; 14A), the ties (26; 26A) are in traction situation when the valve (10; 10A) is in closed configuration, whereas in open configuration the ties (26; 26A) are loose.

2. Valve (10; 10A) in accordance with claim 1, wherein the valve is integrated and coordinated with a pump (50; 50A) of ferromagnetic fluid type.

3. Valve (10; 10A) in accordance with claim 2, wherein the ferromagnetic fluid pump is an artificial heart ventricle (50; 50A).

4. Valve (10) in accordance with claim 1, wherein the closure flaps are three in number (20-22).

5. Valve (10A) in accordance with claim 1, wherein the closure flaps are two in number (20A, 22A).

6. Valve (10; 10A) in accordance with claim 1, wherein the means for generating a magnetic field (M) comprise a winding (16; 16A) of electrically conductive wire through which a direct electric current can be made to pass.

7. Valve (10; 10A) in accordance with claim 6, wherein the winding (16; 16A) is enclosed within the annular element (14; 14A).

8. Valve (10) in accordance with claim 1, wherein the pressure sensor means comprise two pressure sensors (42, 44) located within the annular element (14; 14a), respectively just upstream (42) and just downstream (44) of the closure flaps (20-22).

9. Valve (10) in accordance with claim 1, wherein the control unit (60) is of microprocessor type.

10. Valve (10A) in accordance with claim 1, wherein the ferromagnetic fluid contained in the closure flaps (20A, 22A) of the valve (10A) forms part of the ferromagnetic fluid (24A) of a pump of ferromagnetic fluid type (50A), the closure flaps (20A, 22A) of the valve (10A) forming part of the flexible pocket (30A) containing the ferromagnetic fluid (24A) of the pump (50A).

11. Valve (10; 10A) in accordance with claim 1, wherein the biological liquid is blood.

12. Valve (10; 10A) in accordance with claim 1, wherein the control unit (60) is arranged to anticipate the activation of the magnetic field (M) by the time required for the flaps (20-22; 20A, 22A) to pass from their open condition to their closed condition.

## Patentansprüche

1. Rückschlagventil (10, 10a) für Pumpen (50; 50A) für biologische Flüssigkeiten, umfassend ein im Wesentlichen steifes ringförmiges Element (14; 14A) eines biokompatiblen Materials, welches eine Ventilöffnung und Ventilöffnungsverschlussmittel (18; 18A) ebenfalls aus einem biokompatiblen Material einschließt, wobei die Ventilöffnungsverschlussmittel (18; 18a) mindestens zwei flexible Verschlussklappen (20-22; 20A, 22A) eines biokompatiblen Materials umfassen, welche sich zu dem Inneren der Ventilöffnung erstrecken und welche die Klappen eines natürlichen Herzens nachbilden, wobei vorhanden sind:
- Mittel (16; 16A) zur Erzeugung eines magnetischen Feldes (M);
- im Wesentlichen undehnbare Verbindungselemente (26; 26A) ebenfalls aus einem biokompatiblen Material;
- Drucksensormittel (42, 44), welche ermöglichen, dass ein Druck einer biologischen Flüssigkeit stromaufwärts und stromabwärts des Ventils (10; 10A) gemessen wird;
- eine Steuereinheit (60), um das magnetische Feld (M) zu aktivieren, wenn die Drucksensoren (42, 44) erfassen, dass der Druck in der biologischen Flüssigkeit stromaufwärts und stromabwärts des Ventils (10; 10A) gleich ist und um es spätestens zu deaktivieren, wenn das Ventil (10; 10A) geöffnet wird,
**dadurch gekennzeichnet,**
**dass** die Klappen (20-22; 20A, 22A) in ihrem Inneren, zumindest in ihrem distalen Teil, ein ferromagnetisches Fluid (24; 24A) enthalten,
**dass** die Mittel zur Erzeugung eines magnetischen Feldes bewirken, indem sie auf das ferromagnetische Fluid (24; 24A), welches in den Verschlussklappen (20-22; 20A, 22A) enthalten ist, einwirken, dass diese sich gegenseitig annähern und folglich das Ventil (10; 10A) schließen,
**dass** das distal Ende jeder Klappe (20-22; 20A, 22A) an einem Ende eines entsprechenden Verbindungselements (26; 26A) befestigt ist, wobei das andere Ende des Verbindungselements (26; 26A) an dem Boden des ringförmigen Elements (14; 14A) befestigt ist, wobei sich die Verbindungselemente (26; 26A) in einer Zugsituation befinden, wenn das Ventil (10; 10A) in einer geschlossenen Konfiguration vorliegt, wohingegen die Verbindungselemente (26; 26A) in einer offenen Konfiguration frei sind.

2. Ventil (10; 10A) nach Anspruch 1,
**dadurch gekennzeichnet,**
**dass** das Ventil mit einer Pumpe (50; 50A) eines ferromagnetischen Fluid-Typs integriert oder an diese angepasst ist.

3. Ventil (10; 10A) nach Anspruch 2, **dadurch gekennzeichnet, dass** die Pumpe des ferromagnetischen Fluid-Typs eine künstliche Herzkammer (50; 50A) ist.

4. Ventil (10) nach Anspruch 1, **dadurch gekennzeichnet, dass** die Verschlussklappen drei Verschlussklappen (20-22) sind.

5. Ventil (10A) nach Anspruch 1, **dadurch gekennzeichnet, dass** die Verschlussklappen zwei Verschlussklappen (20A, 22A) sind.

6. Ventil (10; 10A) nach Anspruch 1, **dadurch gekennzeichnet, dass** die Mittel zur Erzeugung eines magnetischen Feldes (M) eine Wicklung (16; 16A) eines elektrisch leitfähigen Drahtes umfassen, durch welchen ein elektrischer Gleichstrom fließen kann.

7. Ventil (10; 10A) nach Anspruch 6, **dadurch gekennzeichnet, dass** die Wicklung (16; 16A) in dem ringförmigen Element (14; 14 A) eingeschlossen ist.

8. Ventil (10) nach Anspruch 1, **dadurch gekennzeichnet, dass** die Drucksensormittel zwei Drucksensoren (42, 44) umfassen, welche innerhalb des ringförmigen Elements (14; 14a) genau stromaufwärts (42) und genau stromabwärts (44) der Verschlussklappen (20-22) angeordnet sind.

9. Ventil (10) nach Anspruch 1, **dadurch gekennzeichnet, dass** die Steuereinheit (60) ein Mikroprozessor-Typ ist.

10. Ventil (10A) nach Anspruch 1, **dadurch gekennzeichnet, dass** das ferromagnetische Fluid, welches in den Verschlussklappen (20A, 22A) des Ventils (10A) enthalten ist, einen Teil des ferromagnetischen Fluids (24A) einer Pumpe eines ferromagnetischen Fluid-Typs (50A) bildet, wobei die Verschlussklappen (20A, 22A) des Ventils (10A) einen Teil der flexiblen Tasche (30A), welche das ferromagnetische Fluid (24A) der Pumpe (50A) enthält, ausbilden.

11. Ventil (10; 10A) nach Anspruch 1, **dadurch gekennzeichnet, dass** die biologische Flüssigkeit Blut ist.

12. Ventil (10; 10A) nach Anspruch 1, **dadurch gekennzeichnet, dass** die Steuereinheit (60) derart ausgestaltet ist, dass sie die Aktivierung des magnetischen Feldes (M) durch die Zeit, welche von den Klappen (20-22; 20A, 22A) benötigt wird, um von ihrer offenen Stellung in ihre geschlossene Stellung zu kommen, antizipiert.

## Revendications

1. Valve antiretour (10, 10a) pour des pompes (50 ; 50A) pour des fluides biologiques, comprenant un élément annulaire (14 ; 14A) sensiblement rigide en matériau biocompatible, qui renferme l'ostium valvulaire, et des moyens de fermeture d'ostium valvulaire (18 ; 18A), également réalisés en de matériau biocompatible, les moyens de fermeture d'ostium valvulaire (18 ; 18a) comprenant au moins deux rabats de fermeture flexibles (20 - 22 ; 20A, 22A) en matériau biocompatible, qui s'étendent vers l'intérieur de l'ostium valvulaire et qui imitent les rabats des valves de coeur naturel, et l'on prévoit :
des moyens (16 ; 16A) pour générer un champ magnétique (M) ;
des attaches (26 ; 26A) sensiblement non extensibles, également en matériau biocompatible ;
des moyens de capteur de pression (42, 44) qui permettent de mesurer la pression du liquide biologique en amont et en aval de la valve (10 ; 10A) ;
une unité de commande (60) pour activer le champ magnétique (M) lorsque les capteurs de pression (42, 44) détectent que les pressions dans le liquide biologique en amont et en aval de la valve (10 ; 10A) sont identiques, et pour le désactiver, au plus tard, lorsque la valve (10 ; 10A) doit être ouverte, **caractérisée en ce que** :
lesdits rabats (20 - 22 ; 20A, 22A) contiennent dans leur intérieur, au moins dans leur partie distale, un fluide ferromagnétique (24 ; 24A) ;
lesdits moyens pour générer un champ magnétique, en agissant sur le fluide ferromagnétique (24 ; 24A) contenu dans les rabats de fermeture (20 - 22 ; 20A, 22A), les amènent à se rapprocher mutuellement et à fermer par conséquent la valve (10 ; 10A) ;
l'extrémité distale de chacun desdits rabats (20 - 22 ; 20A, 22A) est fixée sur une extrémité de ladite attache (26 ; 26A) respective, l'autre extrémité de ladite attache (26 ; 26A) est fixée au fond de l'élément annulaire (14 ; 14A), les attaches (26 ; 26A) étant en situation de traction lorsque la valve (10 ; 10A) est dans la configuration fermée, alors que dans la configuration ouverte, les attaches (26 ; 26A) sont relâchées.

2. Valve (10 ; 10A) selon la revendication 1, dans laquelle la valve est intégrée et coordonnée avec une pompe (50 ; 50A) de type à fluide ferromagnétique.

3. Valve (10 ; 10A) selon la revendication 2, dans laquelle la pompe à fluide ferromagnétique est un ventricule cardiaque artificiel (50 ; 50A).

4. Valve (10) selon la revendication 1, dans laquelle les rabats de fermeture sont au nombre de trois (20 - 22).

5. Valve (10A) selon la revendication 1, dans laquelle les rabats de fermeture sont au nombre de deux (20A, 22A).

6. Valve (10 ; 10A) selon la revendication 1, dans laquelle les moyens pour générer un champ magnétique (M) comprennent un enroulement (16 ; 16A) de fil électriquement conducteur par lequel on peut faire passer un courant électrique continu.

7. Valve (10 ; 10A) selon la revendication 6, dans laquelle l'enroulement (16 ; 16A) est enfermé à l'intérieur de l'élément annulaire (14 ; 14A).

8. Valve (10) selon la revendication 1, dans laquelle les moyens de capteur de pression comprennent deux capteurs de pression (42, 44) situés à l'intérieur de l'élément annulaire (14 ; 14a), respectivement juste en amont (42) et juste en aval (44) des rabats de fermeture (20 - 22).

9. Valve (10) selon la revendication 1, dans laquelle l'unité de commande (60) est de type à microprocesseur.

10. Valve (10A) selon la revendication 1, dans laquelle le fluide ferromagnétique contenu dans les rabats de fermeture (20A, 22A) de la valve (10A) fait partie du fluide ferromagnétique (24A) d'une pompe de type à fluide ferromagnétique (50A), les rabats de fermeture (20A, 22A) de la valve (10A) faisant partie de la pompe flexible (30A) contenant le fluide ferromagnétique (24A) de la pompe (50A).

11. Valve (10 ; 10A) selon la revendication 1, dans laquelle le liquide biologique est du sang.

12. Valve (10 ; 10A) selon la revendication 1, dans laquelle l'unité de commande (60) est agencée pour anticiper l'activation du champ magnétique (M) selon le temps nécessaire pour que les rabats (20 - 22 ; 20A, 22A) passent de leur condition ouverte à leur condition fermée.
